# EUROPEAN PATENT SPECIFICATION

(11) **EP 2 826 473 B1**
(45) Date of publication and mention of the grant of the patent: **19.08.2020**
(21) Application number: 13761828.6
(22) Date of filing: 15.03.2013
(51) Int. Cl.: A61K 31/045, A61P 31/04

(54) **ANTIBACTERIAL USE OF PATCHOULOL**
ANTIBAKTERISCHE VERWENDUNG VON PATCHOULOL
UTILISATION ANTIBACTÉRIENNE DU PATCHOULOL

(30) Priority: 16.03.2012 CN 201210069594
(43) Date of publication of application: 21.01.2015
(73) Proprietor: Chengdu Huasun Technology Group Inc., Ltd., Chengdu Sichuan 610041 (CN); Chengdu University of Traditional Chinese Medicine, Chengdu, Sichuan 611137 (CN)
(72) Inventor: PENG, Cheng, Chengdu Sichuan 611137 (CN); WAN, Feng, Chengdu Sichuan 610041 (CN); XIONG, Liang, Chengdu Sichuan 611137 (CN); LIN, Dasheng, Chengdu Sichuan 610041 (CN); TANG, Zhengwei, Chengdu Sichuan 611137 (CN)
(74) Representative: Epping - Hermann - Fischer
(86) International application number: PCT/CN2013/072660
(87) International publication number: WO 2013/135192

(56) References cited:
- WO-A1-2005/087244
- CN-A- 1 174 724
- CN-A- 101 485 647
- CN-A- 102 349 884
- US-A1- 2006 134 239
- US-A1- 2011 300 243
- EDWARDS-JONES V ET AL: "The effect of essential oils on methicillin-resistant Staphylococcus aureus using a dressing model", BURNS, BUTTERWORTH HEINEMANN, GB, vol. 30, no. 8, 1 December 2004 (2004-12-01), pages 772-777, XP004650469, ISSN: 0305-4179, DOI: 10.1016/J.BURNS.2004.06.006
- DATABASE WPI Week 201152 Thomson Scientific, London, GB; AN 2011-H27692 XP002742523, -& CN 102 058 660 A (UNIV CHENGDU TRADITIONAL CHINESE MEDICIN) 18 May 2011 (2011-05-18)
- CHAUMONT, JEAN-PIERRE: 'ANTIBACTERIAL ACTIVITY ON SKIN AND CHEMICAL COMPOSITION OF THE VOLATILE OILS FROM Agastache rugosa AND Pogostemon cablin' JOURNAL OF MICROBIOLOGY vol. 18, no. 4, December 1998, pages 1 - 4, XP008174793
- HU L F ET AL: "GC-MS fingerprint of Pogostemon cablin in China.", JOURNAL OF PHARMACEUTICAL AND BIOMEDICAL ANALYSIS 18 SEP 2006, vol. 42, no. 2, 18 September 2006 (2006-09-18), pages 200-206, ISSN: 0731-7085
- SWAMY MALLAPPA KUMARA ET AL: "A Comprehensive Review on the Phytochemical Constituents and Pharmacological Activities of Pogostemon cablin Benth.: An Aromatic Medicinal Plant of Industrial Importance.", MOLECULES (BASEL, SWITZERLAND) 12 MAY 2015, vol. 20, no. 5, 12 May 2015 (2015-05-12), pages 8521-8547, ISSN: 1420-3049
- Ngampong Kongkathip ET AL: "Development of Patchouli Extraction with Quality Controland Isolation of Active Compounds with Antibacterial Activity", Kasetsart Journal, vol. 43 1 January 2009 (2009-01-01), pages 519-525, XP055451895, Bangkok, 10900, Thailand Retrieved from the Internet: URL:http://www.thaiscience.info/Journals/A rticle/TKJN/10974308.pdf

## Description

### Field

The disclosure relates to a novel use of patchoulol, belonging to fields of medicaments, healthcare food products, food products, cosmetics, disinfectants or daily supplies, etc.

### Background

Bacterial infectious disease is a systemic infection caused by toxins and other metabolites produced by growth and breeding of pathogenic bacteria or conditional pathogenic bacteria invading blood circulation, and it is always considered as an extremely dangerous disease. Until penicillin was discovered accidently by Alexander Fleming in 1928 and used in clinic, human made a major breakthrough in the battle against infectious diseases. However, with the wide use of antibiotics, the hazard of the antibiotic misuse is far beyond the people's imagination. Methicillin resistant *Staphylococcus aureus* (MRSA) was firstly discovered by Jevons who was from England, and since its discovery, MRSA infections almost spread all over the world, and become one of crucial pathogenic bacteria of nosocomial infections. Besides being methicillin-resistant, the research indicated MRSA was also resistant to other β-lactam antibiotics and cephalosporin antibiotics with similar structures to that of methicillin. By different mechanisms such as changing the target site of antibiotics, producing modification enzyme or lowering membrane permeability, etc, MRSA can develop different resistances to all of aminoglycosides, macrolides, tetracyclines, fluoroquinolones, sulfonamides, rifampicin, *i.e*. has broad spectrum drug resistance, and vancomycin becomes the final first-line drug for overcoming MRSA infections. However, due to the use of vancomycin in the past few years, some examples of vancomycin-resistant *Staphylococcus aureus* (VRSA) were discovered, which made effective control of drug resistance bacterial infections become more difficult.

The occurrence of drug-resistant bacteria let people realize that bacterial drug resistance is developed at a much higher speed compared with the speed of developing new drugs. If this continues, someday, all of the available antibiotics will become inefficient due to the drug tolerance. When people are in a bad shape for the rigorous reality that drug resistant bacteria and organism drug tolerance grow quickly, traditional Chinese medicines occur as new antibacterials and are popular to the majority of patients because of their significant antibacterial property, little toxicity and side-effects.

Patchoulol, also called patchouli alcohol, is a tricyclic sesquiterpene compound that occurs naturally in plants, with a molecular formula of C₁₅H₂₆O and a molecular weight of 222.37. Its chemical name is (1*R*,4*S*,4a*S*,6*R*,8a*S*)-octahydro-4,8a,9,9-tetramethyl-1,6-methanonaphthalen-1 (2H)-ol. Patchoulol is colorless crystal, with light patchouli aroma. It is insoluble in water and freely soluble in petroleum ether, ethanol and other commonly used organic solvents.

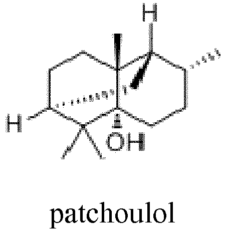

Currently, the investigation on biological activities of patchoulol indicates that it can effectively inhibit the xylene-induced ear edema in mice and carrageenan-induced paw edema in rats, and its activity is dose-dependent. Amongst, high dosage of patchoulol can significantly reduce the content of NO, PGE-2, TNF-α in the hind paw of inflammatory rats, and inhibit the expression of iNOS mRNA^{[1]}. Patchoulol shows significant *in vivo* and *in vitro* anti-influenza virus activity, with an IC₅₀ value of about 2.635 µM against influenza virus A/PR/8/34 (H1N1) *in vitro,* and its activity is dose-dependent; while for its inhibitory on influenza virus type A (H2N2), its IC₅₀ is 4.03±0.23 µM. *In vivo*, patchoulol shows obvious protective effect on model mice infected by influenza virus H2N2 via intragastric administration of 5 mg/(kg·d)^{[2]}; Administration of 30 mg/kg patchoulol can inhibit the pathological change of pneumonia in mice infected by influenza virus FM1, improve the survival rate of mice infected by influenza virus and elongate their survival time; Intragastric administration of 40 and 80 mg/kg patchoulol for successive 7 days can obviously improve the survival rate of nonlethal and lethal model mice infected by influenza virus A/FM/1/47 (H1N1), increase the content of IgA, IgM and IgG in blood serum of model mice, and enhance the numbers of lymphocytes CD3⁺ and CD4⁺, while reduce CD8⁺ numbers. Patchoulol has the functions of regulating humoral immunity and mononuclear phagocyte functions in immunocompromise mice. By intragastric administration of 40 and 80 mg/(kg·d) for successive 7 days, patchoulol can effectively improve thymus gland index, spleen index, sanguinin content, and clearance index in immunocompromise mice induced by prednisone acetate^{[3]}. In addition, patchoulol shows obviously toxic and removal effect on termites^{[4]}; At the dosages of 0.05, 0.5, 5 µg/ml, patchoulol can *in vitro* inhibit the increase of intra-cellular ROS level and [Ca²⁺]_{I} induced by Aβ_{25∼35} belonging to estrogen receptor β-subtype, lessen the apoptosis, possess inhibitory action on neurotoxicity of Aβ_{25∼35}^{[5]}; Administration of 50-70 mg/kg patchoulol can obviously inhibit vomit response of chickens induced by CuSO₄^{[6]}; Intragastric administration of 80 and 40 mg/kg can enhance learning and memory ability of mice with acquired learning and memory disorders induced by scopolamine, obviously lower AchE activity in brain of model mice, improve Chat activity and M1 acceptor level in brain^{[7]}. Safety evaluation studies on patchoulol indicat that intragastrical administration of patchoulol solution prepared in 0.5% CMC-NA to mice at maximum concentration and at maximum dosage volume for successive 14 days shows the maximum tolerated dose of patchoulol for mice is less than 12.5 g/kg^{[8]}. CN1174724 discloses the antibacterial activity of patchouli oil from Radix agastaches comprising 41% patchouli alcohol against various *Staphylococcus spp, including S. epidermidis, S. hominis, S. cohnii, S. haemolyticus, S. xylosus.*

At present, there are no reports on the use of patchoulol for treatment of bacterial infectious diseases.

### Summary

The object of the present disclosure is to provide a novel use of patchoulol.

The present disclosure provides a use of patchoulol in the preparation of antibacterial medicaments, healthcare food products, food products, cosmetics, disinfectants or daily supplies.

Further, said medicicaments, healthcare food products, food products, cosmetics, disinfectants or daily supplies are those medicicaments, healthcare food products, food products, cosmetics, disinfectants or daily supplies used for prevention, treatment or adjuvant therapy of bacterial infections. Said bacterial infections are those caused by pathogenic bacteria or conditional pathogenic bacteria.

Still further, said bacteria are medicament-resistant bacteria.

Further, said bacteria are gram-positive bacteria or gram-negative bacteria.

Further, said gram-positive bacteria are *Staphylococcus spp*., *Enterococcus spp., Streptococcus spp., Kurthia spp., Megasphaera spp., Listeria spp., Erysipelothrix spp., Renibacterium spp., Bacillocin, Clostridium spp., Mycobacterium spp., Actinomyces spp., Nocardia spp., Corynebacterium spp., Rhodococcus spp., Bacillus anthracis, Erysipelothrix, Bacillus tetani, Listeria, Bacillus aerogenes capsulatus, Bacillus gangraenae emphysematosae* or *Tubercle bacillus.*

Further, said gram-negative bacteria are *Proteus vulgaris, Enterobacter cloacae, Chryseobacterium indologenes, Pseudomonas aeruginosa, Acinetobacter lwoffii, Klebsiella pneumoniae,* and *Escherichia coli.*

Still further, said medicament-resistant bacteria are methicillin-resistant bacteria or extended spectrum β-lactamase-producing bacteria.

Further preferably, said medicament-resistant bacteria are methicillin-resistant *Staphylococcus aureus* or methicillin-resistant *Staphylococcus epidermidis.*

Further preferably, said medicament-resistant bacteria are extended spectrum β-lactamase-producing *Escherichia coli* or extended spectrum β-lactamase-producing *Klebsiella pneumoniae.*

Further, said daily supplies are bathroom products or abluents.

Still further, said bathroom products are soap, toothpaste, body wash, hand sanitizer, laundry liquid or mouthwash; said abluents are liquid detergents.

The present disclosure also provides an antibacterial medicament, healthcare food product, food product, cosmetic, disinfectant or daily supplies, characterized in that it is a preparation that is prepared using patchoulil as an active ingredient, together with acceptable excipients or auxiliary ingredients for the fields of antibacterial medicaments, healthcare food products, food products, cosmetics, disinfectants or daily supplies.

Amongst, said preparation is liquid preparation, gas preparation, solid preparation, or semisolid preparation.

Obviously, in light of the above content of present disclosure, according to general technical knowledge and commonly used means in the related field of the present disclosure, many other modifications, replacements or variations can be made, without department from the essential techniques and spirits of the present disclosure.

Patchoulol possesses good inhibitory activities against pathogenic bacteria or conditional pathogenic bacteria, and can be effectively used for treatment of bacterial infectious diseases. Meanwhile, patchoulol is effective against methicillin-resistant bacteria, and its potency is even equivalent to that of vancomycin, providing possibilities for slowing down or avoiding the occurrence of drug-resistant bacteria.

### Detailed Description

**The invention is defined by the appended claims. Any embodiment not falling under the scope of the appended claims does not form part of the invention.**

Patchoulol (abbreviated to PC) used in the present disclosure is all commercially available, and its structure is identified as the following:

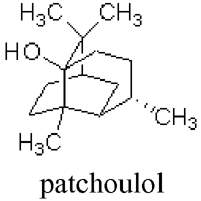

Its purity was determined to be above 98%.

### Example 1 Preparation of medicament granules according to the present disclosure

Patchoulol was added with suitable amount of starch and dextrin, and then granulated to obtain granules.

### Example 2 Preparation of mouthwash according to the present disclosure

Patchoulol was uniformly dispersed with propylene glycol, Tween-60, distilled water, glycerol, surface-active agents, ispropanol, and then filtrated. The filtrate was packaged to obtain a mouthwash.

### Example 3 Preparation of cosmetics according to the present disclosure

Patchoulol was uniformly mixed with suitable amount of lanolin, glycerin monostearate, vaseline, insect white wax, stearic acid, liquid paraffin, benzoic acid, triethanolamine, propylene glycol, distilled water, Tween-60, sodium metabisulfite, essence, preservatives, cerebrin peptide, fruit acid sodium, cetyl alcohol, and then cooled for storage, to obtain a cosmetic.

### Example 4 Preparation of disinfectant according to the present disclosure

Patchoulol was uniformly mixed with Tween-60, carboxymethylcellulose sodium, polyethylene glycol 200 and distilled water to obtain a disinfectant.

### Example 5 Preparation of daily supplies - abluent according to the present disclosure

Patchoulol was mixed with suitable amount of carboxylic acid polymers, nonionic wetting agents, anionic surfactants, enzymes, enzyme stabilizers, foam control agents, Tween-80, and water to prepare an abluent.

### Example 6 Preparation of daily supplies - hand sanitizer according to the present disclosure

Patchoulol was dispersed with glycerol, sodium benzoate, sodium chloride, deionized water, essences, triethanolamine, glyceryl monostearate, hydrogenated lanolin, stearic acid, ethylparaben, mechelie essence, and then can-filled to obtain a hand sanitizer.

### Example 7 Preparation of food product - red babyberry preserves according to the present disclosure

Patchoulol was mixed with suitable amount of orange peel powder, cinnamon powder, granulated sugar, *Flos caryophylli* powder, licorice powder, fennel powder, table salt, and alumen, to prepare red babyberry preserves.

Once red babyberry was prepared well, a layer of 20 cm thick of red babyberry was firstly placed, then a layer of mixed table salt, alumen, and patchoulol was placed, pressed hard. Afterward, a layer of red babyberry followed by a layer of mixture of table salt, alumen, and patchoulol were successively placed and pressed hard, and then pickled to obtain red babyberry base. Red babyberry base was added with granulated sugar, flavor powder, orange peel powder, cinnamon powder, *Flos caryophylli* powder, licorice powder, fennel pulveratus, and then immersed in water, dried in the sun, mixed and packed to obtain red babyberry preserves.

### Example 8 Preparation of healthcare food - hepatoprotective tea according to the present disclosure

Patchoulol was uniformly mixed with suitable amount of carboxymethyl cellulose sodium, and suitable amount of capillary wormwood herb, Villous Amonmum Fruit, medlar, Finger Citron Fruit, *Folium isatidis* fresh tea, chrysanthemum, *Semen cassiae,* honeysuckble, *Atractylodes macrocephala, Cornus officinalis, White Paeony Root,* mulberry leaf, *Schisandra Chinensis, Foeniculum vulgare,* common yam rhizome, to prepare hepatoprotective tea.

Beneficial effects of the present disclosure will be illustrated in detail by the following experimental examples.

### Experimental example 1 Study on the in vitro bacteriostatic activity of patchoulol

Examples which do not fall under the scope of the claims do not form part of the invention.

### 1 Materials and instruments

### 1.1 Laboratory instruments

Multipoint inoculation instrument (Sakuma Manufacturing of Japan, SAKUMA MIT-P type), electric-heated thermostatic water bath (Beijing Tonghua Medical Apparatus and Instruments Factory, DSY-1-6 hole), Laboratory High Pressure Sterilizer (SANYO, MLS-3780 type), CO₂ INCUBATOR (SANYO, MOC-15A), Energy-saving Microbench (Chengdu Feilante Purification Engineering Co. Ltd).

### 1.2 Experimental strains

① Quality control strains: *Staphylococcus aureus* ATCC 29213; *Staphylococcus aureus* ATCC 25923; Methicillin-resistant *Staphylococcus aureus* ATCC 43300; *Staphylococcus epidermidis* ATCC 12228; *Enterococcus faecium* ATCC 33186; *Enterococcus faecalis* ATCC 29212; *Enterococcus faecalis* (high tolerance) ATCC 51299; *Streptococcus pneumoniae* ATCC 49619; *Escherichia coli* ATCC 25922; *Klebsiella pneumoniae* ATCC 700603. All of the quality control strains were provided by Sichuan Industrial Institute of Antibiotics.
② Tested strains: *Staphylococcus aureus* MSSA, *Staphylococcus aureus* MRSA, *Staphylococcus epidermidis* MSSE, *Staphylococcus epidermidis* MRSE, *Enterococcus faecalis, Enterococcus faecium, Escherichia coli, Klebsiella pneumoniae*, *Escherichia coli* producing extended spectrum β-lactamase, *Klebsiella pneumoniae* producing extended spectrum β-lactamase, *Streptococcus pneumoniae*, *Streptococcus agalactiae, Streptococcus uberis, Streptococcus bovis, Corynebacterium, Kurthia gibsonii*, *Megasphaera elsdenii, Staphylococcus wameri, Staphylococcus sciuri, Staphylococcus saprophyticus, Staphylococcus xylosus, Staphylococcus auricularis, Staphylococcus haemolyticus, Proteus vulgaris, Enterobacter cloacae, Chryseobacterium indologenes, Pseudomonas aeruginosa, Acinetobacter lwoffii, Shiglla flexneri,* etc. All of the above strains were clinically isolated pathogenic bacteria which were collected from 3A Grade hospitals in Chengdu of Sichuan province and Beijing from October 2010 to January 2012. Samples mainly were collected from blood, phlegm, urine, etc. After collection, tested strains were identified via BioMeriruk VITEK-32 and VITEK-60 automatic microorganism assay analyzer (France) in collection sites, and further confirmed by Biolog bacterial identification instrument (USA), API 20E, 20NE, Staph series and general methods in Sichuan Industrial Institute of Antibiotics before being used in the experiments.

### 1.3 Drugs

### Tested drug: patchoulol (PC).

**Positive drug:** Azithromycin for injection (Hunan Zhongnan Kelun Pharmaceutical Co., Ltd., batch number: B1103141); Vancomycin hydrochloride for injection (Xinchang Pharmaceutical Factory of Zhejiang Pharmaceutical Co. Ltd, batch number: 111204); Benzylpenicillin sodium for injection (North China Pharmaceutical Co. Ltd, batch number: Y1104101); Cefepime hydrochloride for injection (Huabei Pharmaceutical Kairuite Pharmaceutical Co., Ltd., batch number: B8070803); Levofloxacin hydrochloride injection (Yangtze River Pharmaceutical Group Co., Ltd., batch number: 11121831).

### 1.4 Culture media, reagents and consumables

Mueller-Hinton agar (OXOID, batch number: 729683), nutrient agar culture medium (Hangzhou Microbial Reagent Co., Ltd., batch number: 20100831-02), sodium chloride (Tianjin Damao Chemical Reagent Factory, batch number: 20080328), MILLEX-GP Filter Unit (0.22 µm), disposable sterile petri dish (90 mm, purchased from Jiangsu Kangjian Biotechnology Co., Ltd.), Mcfarland Standard (bioMeriéux, Inc., batch number: 821772701), etc.

### 2 Experimental methods

Preparation of drug-containing flat plates: patchoulol was diluted by coubling double dilution in DMSO, and 1 ml and 14 ml of sterile MH culture media in different gradient concentrations were respectively added to the disposable sterile petri dishes. Tested drugs were respectively diluted to different gradient concentrations from 768 to 0.006 µg/ml, fully mixed, and dried to reserve. As mentioned above, replacing tested drug with same amount of normal saline was used to prepare positive control plates.

Preparation of bacterial liquid: Tested strains and drug-sensitive strains used for quality control were diluted with stroke-physiological saline solution to the concentration of 10⁶ CFU/ml.

Bacteriostatic activity assay *in vivo*: using multipoint inoculation instrument, the tested strain liquid was added to the drug-containing flat plates with different gradient concentrations and positive control plates respectively, and replacing the bacterial liquid with 5 µL normal saline, served as the negative control.

*Staphylococcus aureus* and *Staphylococcus epidermidis* were cultured at 35-37°C for 18-24 h in MH media (containing 2% NaCl); *Streptococcus pneumonia* was cultured in brain heart infusion agar media (containing 5% goat blood) within an incubator under 5% CO₂ at 35°C for 24 hours; Gram-negative bacteria and other strains were placed in an incubator at 35-37°C and cultured for 18-20 h; Results were observed and recorded.

Result assessment: The minimum concentration of drug in no bacterial growth plates was regarded as the minimum inhibitory concentration (MIC) of the drug to the stain.

### 3. Experimental results

The results of bacteriostatic activity assay of the tested Gram-positive bacteria and Gram-negative bacteria *in vitro* were shown in Tables 1-5.
In which, MSSA: Methicillin-sensitive *Staphylococcus aureus;* MRSA: Methicillin-resistant *Staphylococcus aureus*;
MSSE: Methicillin-sensitive *Staphylococcus epidermidis*; MRSE: Methicillin-resistant Staphylococcus *epidermidis*;
*Escherichia coli* E⁻ : non-ESBLs strains; *Escherichia coli* E⁺: ESBLs strains;
*Klebsiella pneumonia* E⁻ : non-ESBLs strains; *Klebsiella pneumonia* E⁺ : ESBLs strains;
ESBLs: strains producing extended spectrum β-lactamase.

**Table 1**

| Classification | Tested strains (Strain number) | MIC (µg/ml) | Bacterium control (+/-) |
|---|---|---|---|
| | | PC | |
| | *Streptococcus agalactiae* 12-1 | 50 | + |
| | *Streptococcus uberis* 12-1 | 25 | + |
| | *Streptococcus bovis* 12-3 | 50 | + |
| | *Coryneba cterium* 12-1 | 12.5 | + |
| | *Kurthia gibsonii* 12-1 | 50 | + |
| | *Kurthia gibsonii* 12-2 | 50 | + |
| | *Megasphaera elsdenii* 12-1 | 50 | + |
| | *Megasphaera elsdenii* 12-2 | 25 | + |
| | *Megasphaera elsdenii* 12-3 | 50 | + |
| Gram-positive bacteria | *Megasphaera elsdenii* 12-4 | 50 | + |
| | *Staphylococcus wameri* | 50 | + |
| | *Staphylococcus sciuri* 12-1 | 50 | + |
| | *Staphylococcus* saprophyticus 12-1 | 100 | + |
| | *Staphylococcus* saprophyticus 12-2 | 100 | + |
| | *Staphylococcus* saprophyticus 12-3 | 100 | + |
| | *Staphylococcus xylosus* 12-1 | 100 | + |
| | *Staphylococcus xylosus* 12-1 | 100 | + |
| | *Staphylococcus auricularis* | 25 | + |
| | *Staphylococcus auricularis* | 50 | + |
| | *Staphylococcus haemolyticus* | 100 | + |
| Gram-negative bacteria | *Proteus vulgaris* 12-1 | 100 | + |
| | *Enterobacter cloacae* 12-1 | 50 | + |
| | *Enterobacter cloacae* 12-2 | 50 | + |
| | *Chryseobacterium* indologenes 12- | 25 | + |
| | *Chryseobacterium* indologenes 12- | 100 | + |
| | *Pseudomonas aeruginosa* 12-1 | 50 | + |
| | *Acinetobacter lwoffii* 12-1 | 50 | + |
| | *Acinetobacter lwoffii* 12-2 | 50 | + |
| | *Shiglla flexneri* 12-1 | 400 | + |
| | *Shiglla flexneri* 12-2 | 50 | + |

**Table 2**

| Tested strains (Strain number) | MIC (µg/ml) | | | | | | Bacterium control (+/-) |
|---|---|---|---|---|---|---|---|
| | PC | vancomycin | penicillin | azithromycin | cefepime | levofloxacin | |
| *Staphylococcus aureus* MSSA 12-11 | 128 | 1 | 0.5 | >128 | 4 | 0.125 | + |
| *Staphylococcus aureus* MSSA 12-13 | 128 | 1 | 0.5 | 0.25 | 4 | 0.125 | + |
| *Staphylococcus aureus* MSSA 12-14 | 64 | 1 | 0.5 | >128 | 2 | 0.125 | + |
| *Staphylococcus aureus* MSSA 12-15 | 128 | 1 | 0.25 | 0.125 | 2 | 0.125 | + |
| *Staphylococcus aureus* MSSA 12-16 | 64 | 1 | 0.5 | >128 | 4 | 0.06 | + |
| *Staphylococcus aureus* MSSA 12-17 | 64 | 1 | 0.125 | >128 | 4 | 0.06 | + |
| *Staphylococcus aureus* MSSA 12-18 | 128 | 1 | 0.25 | 128 | 2 | 0.125 | + |
| *Staphylococcus aureus* MSSA 12-19 | 128 | 1 | 0.25 | >128 | 2 | 0.06 | + |
| Staphylococcus aureus MSSA 12-20 | 64 | 1 | 0.5 | >128 | 2 | 0.125 | + |
| Staphylococcus aureus MSSA 12-21 | 64 | 1 | 0.25 | 128 | 4 | 0.125 | + |
| *Staphylococcus epidermidis* MSSE 12-11 | 32 | 1 | 0.03 | 128 | 1 | 0.125 | + |
| *Staphylococcus epidermidis* MSSE 12-12 | 32 | 2 | 0.06 | 4 | 1 | 0.125 | + |
| *Staphylococcus epidermidis* MSSE 12-13 | 64 | 1 | 0.5 | >128 | 1 | 0.5 | + |
| *Staphylococcus epidermidis* MSSE 12-14 | 64 | 2 | 8 | 8 | 16 | 0.5 | + |
| *Staphylococcus epidermidis* MSSE 12-15 | 64 | 2 | 8 | 8 | 16 | 0.5 | + |
| *Staphylococcus epidermidis* MSSE 12-16 | 32 | 0.5 | 0.06 | 64 | 0.25 | 0.125 | + |
| *Staphylococcus epidermidis* MSSE 12-17 | 32 | 0.5 | 0.03 | 8 | 2 | 0.5 | + |
| *Staphylococcus epidermidis* MSSE 12-18 | 32 | 0.25 | 0.06 | 16 | 1 | 1 | + |
| *Staphylococcus epidermidis* MSSE 12-19 | 32 | 1 | 0.06 | 16 | 1 | 0.5 | + |
| *Staphylococcus epidermidis* MSSE 12-20 | 64 | 1 | 0.03 | 128 | 2 | 1 | + |
| *Enterococcus faecalis* 12-11 | >128 | >128 | >128 | >128 | >128 | 16 | + |
| *Enterococcus faecalis* 12-12 | 32 | 1 | 1 | 4 | 4 | 32 | + |
| *Enterococcus faecalis* 12-13 | 128 | 2 | 32 | >128 | >128 | 8 | + |
| *Enterococcus faecalis* 12-14 | 64 | 1 | 1 | 1 | 8 | 64 | + |
| *Enterococcus faecalis* 12-17 | 128 | 2 | 2 | >128 | 32 | 0.5 | + |
| *Enterococcus faecalis* 12-18 | 128 | 1 | 2 | >128 | 16 | 0.5 | + |
| *Enterococcus faecalis* 12-19 | 128 | 2 | 2 | >128 | 16 | 32 | + |
| *Enterococcus faecalis* 12-20 | 128 | 2 | 2 | >128 | 16 | 4 | + |
| *Enterococcus faecalis* 12-21 | 64 | 1 | 2 | >128 | 16 | 4 | + |
| *Enterococcus faecalis* 12-22 | 64 | 1 | 1 | >128 | 32 | 8 | + |
| *Enterococcus faecium* 12-7 | 64 | 0.5 | 128 | >128 | >128 | 32 | + |
| *Enterococcus faecium* 12-8 | 64 | 0.5 | >128 | 1 | >128 | 64 | + |
| *Enterococcus faecium* 12-9 | 64 | 1 | 128 | >128 | >128 | 32 | + |
| *Enterococcus faecium* 12-10 | 32 | 4 | >128 | >128 | >128 | 32 | + |
| *Enterococcus faecium* 12-11 | 32 | 0.5 | 128 | >128 | >128 | 32 | + |
| *Enterococcus faecium* 12-12 | 64 | 1 | >128 | >128 | >128 | 64 | + |
| *Enterococcus faecium* 12-13 | 64 | 4 | >128 | >128 | >128 | 64 | + |
| *Enterococcus faecium* 12-14 | 64 | 0.5 | >128 | >128 | >128 | 32 | + |
| *Enterococcus faecium* 12-15 | 32 | 1 | 128 | >128 | >128 | 32 | + |
| *Enterococcus faecium* 12-16 | 32 | 1 | 128 | >128 | >128 | 64 | + |
| *Staphylococcus epidermidis* ATCC 12228 | 64 | 1 | 0.5 | 0.125 | 1 | 0.125 | + |
| *Enterococcus faecium* ATCC 33186 | 128 | 1 | 2 | 2 | 16 | 1 | + |
| *Enterococcus faecalis* ATCC 29212 | 128 | 2 | 1 | 4 | 16 | 0.25 | + |
| *Enterococcus faecalis* (high endurance) ATCC 51299 | 128 | 2 | 1 | >128 | 32 | 0.25 | + |

**Table 3**

| Tested strains (Strain number) | MIC (µg/ml) | | Bacterium control (+/-) |
|---|---|---|---|
| | PC | vancomycin | |
| *Staphylococcus aureus* MRSA 12-11 | 128 | 2 | + |
| *Staphylococcus aureus* MRSA 12-12 | 128 | 1 | + |
| *Staphylococcus aureus* MRSA 12-13 | 64 | 1 | + |
| *Staphylococcus aureus* MRSA 12-14 | 64 | 1 | + |
| *Staphylococcus aureus* MRSA 12-15 | 64 | 1 | + |
| *Staphylococcus aureus* MRSA 12-16 | 64 | 2 | + |
| *Staphylococcus aureus* MRSA 12-17 | 128 | 1 | + |
| *Staphylococcus aureus* MRSA 12-18 | 128 | 1 | + |
| *Staphylococcus aureus* MRSA 12-19 | 64 | 2 | + |
| *Staphylococcus aureus* MRSA 12-20 | 128 | 1 | + |
| *Staphylococcus epidermidis* MRSE 12-11 | 128 | 8 | + |
| *Staphylococcus epidermidis* MRSE 12-12 | 32 | 0.5 | + |
| *Staphylococcus epidermidis* MRSE 12-15 | 32 | 2 | + |
| *Staphylococcus epidermidis* MRSE 12-16 | 32 | 2 | + |
| *Staphylococcus epidermidis* MRSE 12-17 | 32 | 2 | + |
| *Staphylococcus epidermidis* MRSE 12-18 | 32 | 2 | + |
| *Staphylococcus epidermidis* MRSE 12-19 | 32 | 2 | + |
| *Staphylococcus epidermidis* MRSE 12-20 | 128 | 2 | + |
| *Staphylococcus epidermidis* MRSE 12-21 | 128 | 2 | + |
| *Staphylococcus epidermidis* MRSE 12-22 | 32 | 1 | + |
| *Staphylococcus aureus* ATCC 29213 | 64 | 0.5 | + |
| Methicillin-resistant *Staphylococcus aureus* ATCC 43300 | 128 | 1 | + |

**Table 4**

| Tested strains (Strain number) | MIC (µg/ml) | | | Bacterium control |
|---|---|---|---|---|
| | PC | penicillin | levofloxacin | |
| *Streptococcus pneumoniae* 12-1 | 200 | 0.1 | 12.5 | + |
| *Streptococcus pneumoniae* 12-2 | 100 | 0.2 | 25 | + |
| *Streptococcus pneumoniae* 12-3 | 200 | 0.1 | 25 | + |
| *Streptococcus pneumoniae* 12-4 | 200 | 0.4 | 25 | + |
| *Streptococcus pneumoniae* 12-5 | 100 | 0.05 | 25 | + |
| *Streptococcus pneumonia* 12-6 | 100 | 0.05 | 25 | + |
| *Streptococcus pneumonia* 12-7 | 200 | 0.1 | 12.5 | + |
| *Streptococcus pneumonia* 12-8 | 100 | 0.1 | 25 | + |
| *Streptococcus pneumonia* 12-9 | 100 | 0.1 | 25 | + |
| *Streptococcus pneumonia* 12-10 | 200 | 0.2 | 12.5 | + |
| *Streptococcus pneumonia* ATCC 49619 | 200 | 0.2 | 0.1 | + |

**Table 5**

| Tested strains (Strain number) | MIC (µg/ml) | | Bacterium control (+/-) |
|---|---|---|---|
| | PC | levofloxacin | |
| *Staphylococcus aureus* MSSA 10-38 | 1.5 | 0.1 | + |
| *Staphylococcus aureus* MSSA 10-40 | 1.5 | 0.1 | + |
| *Staphylococcus aureus* MSSA 10-41 | 1.5 | 0.1 | + |
| *Staphylococcus aureus* MRSA 10-31 | 1.5 | 25 | + |
| *Staphylococcus aureus* MRSA 10-32 | 1.5 | 12.5 | + |
| *Staphylococcus epidermidis* MSSE 10-43 | 1.5 | 0.1 | + |
| *Staphylococcus epidermidis* MSSE 10-44 | 1.5 | 3.1 | + |
| *Staphylococcus epidermidis* MRSE 10-40 | 1.5 | 0.1 | + |
| *Staphylococcus epidermidis* MRSE 10-44 | 1.5 | 3.1 | + |
| *Escherichia coli* E⁻ 10-12 | >768 | 0.1 | + |
| *Escherichia coli* E⁻ 10-18 | >768 | 12.5 | + |
| *Escherichia coli* E⁺ 10-7 | >768 | 12.5 | + |
| *Escherichia coli* E⁺ 10-9 | >768 | 0.1 | + |
| *Klebsiella pneumonia* E⁻ 10-16 | >768 | 0.025 | + |
| *Klebsiella pneumonia* E⁻ 10-18 | >768 | 0.025 | + |
| *Klebsiella pneumonia* E⁺ 10-2 | >768 | 12.5 | + |
| *Klebsiella pneumonia* E⁺ 10-6 | >768 | 0.0125 | + |
| *Staphylococcus aureus* ATCC 25923 | 1.5 | 0.1 | + |
| *Escherichia coli* ATCC 25922 | 768 | <0.006 | + |
| *Klebsiella pneumonia* ATCC 700603 | >768 | 0.39 | + |

It is seen from Tables 1-5, patchoulol possesses strong bacteriostatic activities against both Gram-positive bacteria and Gram-negative bacteria (methicillin-sensitive *Staphylococcus aureus,* methicillin-resistant *Staphylococcus aureus,* methicillin-sensitive *Staphylococcus epidermidis,* methicillin-resistant *Staphylococcus epidermidis, Enterococcus faecalis, Enterococcus faecium, Streptococcus pneumonia, Streptococcus agalactiae, Streptococcus uberis, Streptococcus bovis, Coryneba cterium, Kurthia gibsonii, Megasphaera elsdenii, Staphylococcus wameri, Staphylococcus sciurim, Staphylococcus saprophyticus, Staphylococcus xylosus, Staphylococcus auricularis, Staphylococcus haemolyticus, Proteus vulgaris, Enterobacter cloacae, Chryseobacterium indologenes, Pseudomonas aeruginosa, Acinetobacter lwoffii, Shiglla flexneri,* etc.), with MIC values of 1.5-200 µg/ml; its MIC values against *Escherichia coli* ESBLs⁻/ESBLs⁺ and *Klebsiella pneumonia* ESBLs⁻/ESBLs⁺ are more than or equal to 768 µg/ml.

The above results indicate that patchoulol has good antibacterial activities against the strains sensitive or drug-resistant to Gram-positive bacteria and Gram-negative bacteria, in which patchoulol obviously inhibits methicillin-resistant bacteria.

### Experimental example 2 In vivo antibacterial activity of patchoulol

### 1 Experimental materials (Reference example)

### 1.1 drugs

Test drug, patchoulol (PC);
Positive drug, vancomycin hydrochloride for injection, specifications: 5×10⁵ units, Xinchang Pharmaceutical Factory of Zhejiang Pharmaceutical Co. Ltd, batch number: 111204;
Drug for making model: Mucin form Porcine Stomach (Type II), specification: 100 g, SIGMA-ALDRICH, batch number: 018K0079.

### 1.2 Strains

Methicillin-resistant *Staphylococcus aureus* ATCC 43300, provided by Sichuan Industrial Institute of Antibiotics.

### 1.3 Animals

KM mice, grade SPF, half male and half female, weighing (20±2) g, provided by Sichuan Institute of Biological Products, certification number SCXK (Chuan) 2008-08.

### 2 Experimental contents

### 2.1 Preliminary test

Tested strains' virulence assay: Methicillin-resistant *Staphylococcus aureus* was cultured in incubator at 37°C to reach exponential phase of growth, and then diluted with normal saline to obtain different gradient concentrations of bacterial liquid. Different concentrations of bacterial liquid were taken and mixed with equivalent 10% gastric mucin. Bacterial liquid was administrated to mice at 0.5 ml/20 g by intraperitoneal injection, and 5% gastric mucin was used as control. Under a premise of no control mice death, the death rate of mice within 14 days was recorded, and the minimum bacterial volumes leading to death of whole mice were measured, i.e. the minimum lethal dose (MLD) of bacterial liquid.

Pretesting of effective dose *in vivo*: Infection model mice infected by Methicillin-resistant *Staphylococcus aureus* were randomly divided into a few of groups (four mice per group), and a graded dose of each bacterial liquid was separately administrated to mice in each test group at one time per day. Moreover, administration of test drug was done one day ahead of schedule, and toxic substances were not eliminated until 0.5 h after administration on the second day. The death status of mice within 14 days was observed, to determine the dosage used in the formal experiment.

### 2.2 In vivo antibacterial activity

KM mice (grade SPF), half male and half female, weighing 18-22 g were randomly divided into eight groups with ten mice for each group, i.e. the group receiving vancomycin hydrochloride for injection; the groups receiving high, medium, and low doses of patchoulol; blank group; model group; and control group receiving gastric mucin. All of the mice were administrated by intraperitoneal injection once a day. The mice were administrated one day in advance and then attacked after 0.5h of administration on the second day, i.e. intraperitoneally injected with methicillin-resistant *Staphylococcus aureus* liquid containing 5% gastric mucin. Amongst, mice in the model group were given normal saline instead of test drug; mice in the blank group were not given drugs and attacked; mice in the gastric mucin control group were not given test drug, but only injected with 5% gastric mucin without bacteria.

### 2.3 Statistical analysis

The death status of mice within 14 days after injection of bacteria was observed, and its survival rate was recorded.

### 3 Experimental results

### 3.1 Preliminary test

Results of preliminary test shows MLD of mice against methicillin-resistant *Staphylococcus aureus* is 1.28×10⁷ CFU/g; high, medium, and low doses of patchouli alcohol used in *in vivo* antibacterial test in mice is determined as 200, 100, and 50 mg/kg, respectively.

### 3.2 In vivo antibacterial activity

Experimental results are shown in the following table.

Table 6 *In vivo* protection of patchoulol on model mice infected with methicillin-resistant *Staphylococcus aureus*

| Group | Animals | dose (mg/kg) | Effect | |
|---|---|---|---|---|
| | | | Survival number | Survial rate (%) |
| Blank | 10 | - | 10 | 100 |
| Model | 10 | - | 0 | 0 |
| Gastric mucin Control | 10 | 1.25 | 10 | 100 |
| Vancomycin Hydrochloride for injection | 10 | 4×10⁵ units/kg | 10 | 100 |
| High dose | 10 | 200 | 10 | 100 |
| Medium dose | 10 | 100 | 10 | 100 |
| Low dose | 10 | 50 | 8 | 80 |

It is seen from the above Table 6 that intraperitoneal injection of patchoulol shows good *in vivo* protective effect on mice infected by methicillin-resistant *Staphylococcus aureus,* and the protective effect on mice in groups of high and medium doses achieves 100%, while the protective effect on mice in low dose group achieves 80%, equal to that of vancomycin hydrochloride for injection.

### Experimental example 3 Prevention effect of patchoulol on bacterial infections (Reference example)

### 1 Experimental materials

### 1.1 Drug

Test drug, patchoulol (PC);

### 1.2 Strains

Methicillin-resistant *Staphylococcus aureus* ATCC 43300, *Staphylococcus aureus* ATCC 25923, provided by Sichuan Industrial Institute of Antibiotics.

### 2 Experimental methods

As representative bacteria, two strains of bacteria mentioned in "Experimental materials" were used in the experiment.

Patchoulol was accurately weighed, and then suitable amount of Tween-60, carboxymethylcellulose sodium, glycerol, and absolute alcohol were added and stirred thoroughly. The mixture was coated on inner wall of tubes, in which the concentrations of patchoulol on inner wall of tubes were 1200, 800, 600, 400, 200, 100, 50, 25, and 0 µg/ml, respectively. After natural drying, tubes were placed in PBS buffer solution to fix for 20 min, and then the procedures were repeated for several times, to form a membrane on inner wall of tubes.

0.1 ml of bacterial suspensions of methicillin-resistant *Staphylococcus aureus* ATCC 43300 or *Staphylococcus aureus* ATCC 25923 was inoculated to sterile cup containing 4.9 ml of normal saline and 1 cm tubes (tubes being horizontally placed), and cultured at 37°C. The liquid was replaced every other day, and after being cultured for successive 7 days, bacteria on the membranes of tubes were rinsed, and the colony count was carried out, eight samples were prepared for each group.

### 3 Experimental results

When concentrations of patchoulol on intraductal membranes are more than or equal to 600 µg/ml, bacterial colony numbers of both *Staphylococcus aureus* and methicillin-resistant *Staphylococcus aureus* are 0; while at the concentrations of 400, 200, 100 and 50 µg/ml, bacterial colony numbers of both bacterial strains decrease, as compared with 0 µg/ml.

In summary, patchoulol possesses good inhibitory activities against pathogenic bacteria and conditional pathogenic bacteria, and can be effectively used for prevention and treatment of bacterial infectious diseases. Meanwhile, patchoulol also can effectively inhibit methicillin-resistant bacteria, and its potency is even equivalent to that of vancomycin, providing possibilities for slowing down or avoiding the occurrence of drug resistance bacteria.

### References

1. Li YC, Xian YF, Ip SP, et al. Anti-inflammatory activity of patchouli alcohol isolated from Pogostemonis Herba in animal models[J]. Fitoterapia, 2011, 82(8): 1295-1301.
3. Wu H, Li B, Wang X, et al. Inhibitory effect and possible mechanism of action of patchouli alcohol against influenza A (H2N2) virus[J].Molecules, 2011,16(8):6489-6501.
4. Peng SZ. Study on screening and assessment of active constituents against influenza virus a in Pogostemon cablin. Guangzhou University of Chinese Medicine, 2011, doctorial dissertation.
5. Zhu BC, Henderson G, Yu Y, et al. Toxicity and repellency of patchouli oil and patchouli alcohol against Formosan subterranean termites Coptotermes formosanus Shiraki (Isoptera: Rhinotermitidae) [J]. J Agric Food Chem, 2003, 51(16):4585-4588.
6. Huang XW, Bai L, Xu FH, etc. Inhibitory effect of Patchouli alcohol on neurotoxicity of β-amyloid. Pharm. J. Chin. PLA [J], 2008, 24 (4): 338-340.
7. Yang Y, Kinoshita K, Koyama K, et al. Anti-emetic principles of Pogostemon cablin (Blanco) Benth [J]. Phytomedicine, 1999, 6(2): 89-93.
8. Huang XW, Liu RT, Lv QJ. Effect of patchouli alcohol on learning and memory function of mice with memory disorders induced by scopolamine [J]. Chin. Tradit. Herb. Drugs, 2009, 40 (9): 1431-1433.
9. Ren SZ, Zhang JQ, Liu MS. Acute toxicity testing of patchouli alcohol [J]. Acta Acad. Med. Hainan, 2009, 15(12): 1503-1504.

## Claims

1. Non-medical use of patchoulol in food products, cosmetics, disinfectants or daily supplies for the prevention of bacterial growth, wherein patchoulol is the only active ingredient and consists of more than 98% of a compound having a structure as (I) showed, and wherein the bacteria is *methicillin-resistant Staphylococcus epidermidis,*

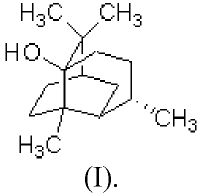

2. The use according to claim 1, **characterized in that** said daily supplies are bathroom products or abluents.

3. The use according to claim 2, **characterized in that** said bathroom products are soap, toothpaste, body wash, hand sanitizer, laundry liquid or mouthwash, and said abluents are liquefied detergent.

4. An antibacterial medicament or healthcare food product comprising patchoulol as the only active ingredient together with acceptable excipients or auxiliary ingredients for use in the prevention, treatment or adjuvant therapy of bacterial infections, wherein patchoulol consists of more than 98% of a compound having a structure as (I) showed, and wherein the bacteria is *methicillin-resistant Staphylococcus epidermidis,*

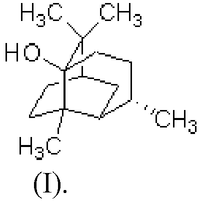

5. The antibacterial medicament or healthcare food product for use of claim 4, wherein said medicament or food product is a liquid preparation, gas preparation, solid preparation or semisolid preparation.

## Patentansprüche

1. Nicht-medizinische Verwendung von Patschuli-Alkohol in Nahrungsmittelprodukten, Kosmetika, Desinfektionsmitteln oder täglichen Verbrauchsgütern zur Verhinderung von Bakterienwachstum, wobei der Patschuli-Alkohol der alleinige Wirkstoff ist und zu mehr als 98% aus einer Verbindung mit einer Struktur wie bei (I) gezeigt besteht, und wobei es sich bei dem Bakterium um den *Methicillin-resistenten Staphylococcus epidermidis* handelt,

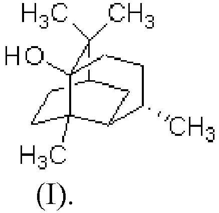

2. Verwendung nach Anspruch 1, **dadurch gekennzeichnet, dass** es sich bei den täglichen Verbrauchsgütern um Badezimmerprodukte oder Reinigungsmittel handelt.

3. Verwendung nach Anspruch 2, **dadurch gekennzeichnet, dass** es sich bei den Badezimmerprodukten um Seife, Zahnpasta, Duschgel, Handdesinfektionsmittel, Waschflüssigkeit oder Mundwasser und bei den Reinigungsmitteln um Flüssigwaschmittel handelt.

4. Antibakterielles Medikament oder Nahrungsmittelprodukt für die Gesundheitspflege, umfassend Patschuli-Alkohol als alleinigen Wirkstoff zusammen mit akzeptablen Trägerstoffen oder zusätzlichen Hilfsstoffen zur Verwendung bei der Vorbeugung, Behandlung oder unterstützenden Therapie von bakteriellen Infektionen, wobei der Patschuli-Alkohol zu mehr als 98% aus einer Verbindung mit einer Struktur wie bei (I) gezeigt besteht, und wobei es sich bei dem Bakterium um den *Methicillin-resistenten Staphylococcus epidermidis* handelt.

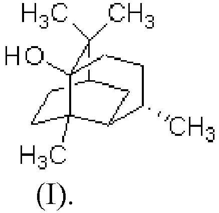

5. Antibakterielles Medikament oder Nahrungsmittelprodukt für die Gesundheitspflege zur Verwendung nach Anspruch 4, wobei das Medikament oder das Nahrungsmittelprodukt eine flüssige Zubereitung, gasförmige Zubereitung, feste Zubereitung oder halbfeste Zubereitung ist.

## Revendications

1. Utilisation non médicale du patchoulol dans des produits alimentaires, des produits cosmétiques, des désinfectants ou des fournitures quotidiennes pour la prévention de la croissance bactérienne, dans laquelle le patchoulol est le seul ingrédient actif et consiste en plus de 98 % d'un composé ayant une structure telle que (I) montrée, et dans laquelle la bactérie est le *Staphylococcus epidermidis résistant à la méthicilline,*

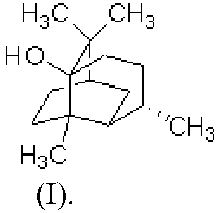

2. Utilisation selon la revendication 1, **caractérisée en ce que** lesdites fournitures quotidiennes sont des produits pour la salle de bains ou des agents de nettoyage.

3. Utilisation selon la revendication 2, **caractérisée en ce que** lesdits produits pour la salle de bains sont du savon, du dentifrice, du gel douche, du désinfectant pour les mains, de la lessive ou du bain de bouche, et lesdits agents de nettoyage sont des détergents liquéfiés.

4. Médicament antibactérien ou produit alimentaire de santé comprenant du patchoulol comme seul ingrédient actif ainsi que des excipients acceptables ou ingrédients auxiliaires pour une utilisation dans la prévention, le traitement ou la thérapie adjuvante des infections bactériennes, dans lequel le patchoulol consiste en plus de 98% d'un composé ayant une structure telle que (I) montrée, et dans lequel la bactérie est le *Staphylococcus epidermidis résistant à la méthicilline,*

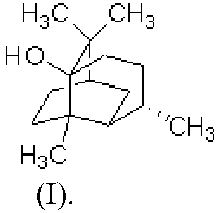

5. Médicament antibactérien ou produit alimentaire de santé pour l'utilisation de la revendication 4, dans lequel ledit médicament ou produit alimentaire est une préparation liquide, une préparation gazeuse, une préparation solide ou une préparation semi-solide.
